## Europäisches Patentamt
## European Patent Office
## Office européen des brevets

(19)

(11) Veröffentlichungsnummer: **0 159 043**
**B1**

(12) # EUROPÄISCHE PATENTSCHRIFT

(45) Veröffentlichungstag der Patentschrift:
20.07.88

(51) Int. Cl.⁴: **A 61 N  1/42**

(21) Anmeldenummer: **85104738.1**

(22) Anmeldetag: **19.04.85**

(54) **Vorrichtung zur Herstellung pulsierender Magnetfelder für therapeutische Zwecke.**

(30) Priorität: **19.04.84  DE 3414938**

(43) Veröffentlichungstag der Anmeldung:
**23.10.85 Patentblatt 85/43**

(45) Bekanntmachung des Hinweises auf die Patenterteilung:
**20.07.88 Patentblatt 88/29**

(84) Benannte Vertragsstaaten:
**AT CH DE FR GB IT LI**

(56) Entgegenhaltungen:
**EP-A-0 040 053**
**DE-A-3 221 544**
**FR-A-1 073 155**
**FR-A-2 210 420**

(73) Patentinhaber: **Rau, Dieter, Wittinger- Weg 15,
D-7347 Bad Überkingen (DE)**
Patentinhaber: **Hörl, Alois, Kopernikusstrasse 1/III,
D-8000 München 80 (DE)**

(72) Erfinder: **Hörl, Alois, Kopernikusstrasse 1/III,
D-8000 München 80 (DE)**

(74) Vertreter: **Wilhelms, Rolf E., Dr., WILHELMS,
KILIAN & PARTNER Patentanwälte Eduard-
Schmid- Strasse 2, D-8000 München 90 (DE)**

EP 0 159 043 B1

LIBER, STOCKHOLM 1988

**Beschreibung**

Die Erfindung betrifft eine Vorrichtung zur Herstellung pulsierender Magnetfelder für therapeutische Zwecke gemäß Oberbegriff des Patentanspruchs 1.

Aus der französischen Patentschrift Nr. 324 317 und ihrer Zusatzpatentschrift 1703 ist eine Vorrichtung bekannt, bei welcher ein hufeisenformiger Magnet um seine zu seinen Schenkeln parallele Achse gedreht wird. Dieser bekannte Aufbau hat radiale Symmetrie, d.h. es gibt mindestens eine die Drehachse enthaltende Ebene, bezüglich der die Polflächen symmetrisch angeordnet sind. Die über der Ebene der Polflächen als Folge einer Drehung des Magneten auftretenden radialen Kräfte sind dann völlig unabhängig von der Drehrichtung des Magneten.

Aus der österreichischen Patentschrift 354 621 ist es bekannt, auf einer Scheibe vier Gruppen von Magneten mit zur Drehachse der Scheibe paralleler Polarisation vorzusehen, wobei jede Gruppe von Magneten praktisch einen 90°-Sektor der Scheibe beansprucht und die Polarisationsrichtungen benachbarten Gruppen entgegengesetzt sind. Auch diese Anordnung ist radialsymmetrisch und zeigt keinerlei Abhängigkeit der von und zur Drehachse gerichteten Kräfte von der Drehrichtung der Scheibe.

Aus der Fr-A-2 210 420 ist eine Vorrichtung zur Herstellung pulsierender Magnetfelder mit einer asymmetrischern Anordnung von Magneten bekannt.

Aufgabe der Erfindung ist die Schaffung einer Vorrichtung zur Erzeugung pulsierender Magnetfelder, welche zur Verbesserung der therapeutischen Wirksamkeit abhängig von der Drehrichtung radial nach innen oder außen gerichtete Kräfte ausüben kann.

Diese Aufgabe wird erfindungsgemäß durch eine Vorrichtung gelöst, wie sie in Patentanspruch 1 gekennzeichnet ist.

Vorteilhafte Weiterbildungen der Erfindung sind Gegenstand der Unteransprüche.

Im folgenden werden bevorzugte Ausführungsformen der Erfindung anhand der beigefügten Zeichnungen beschrieben. Auf diesen zeigt bzw. zeigen

| | |
|---|---|
| Fig. 1 | eine erste Ausführungsform der Vorrichtung schematisch in der Draufsicht, |
| Fig. 2 | eine zweite Ausfhurungsform der Vorrichtung in der Draufsicht, |
| Fig. 3 und 4 | Schnitte längs Linie C-D in Fig. 2 bei verschiedenen Ausführungsformen des Antriebs, |
| Fig. 5 | eine dritte Ausführungsform der Vorrichtung in einer der Fig. 2 entsprechenden Ansicht, |
| Fig. 6 und 7 | Schnitte längs Linie C-D der |

Fig. 5 bei verschiedenen Ausführungsformen des Antriebs,

| | |
|---|---|
| Fig. 8 und 9 | schematische Darstellungen, die wiedergeben, wie sich ferromagnetisches Granulat auf einer amagnetischen Platte über der Magnetanordnung gemäß den Figuren 1 und 2 bei Rechtsdrehung und Linksdrehung des Magnetträgers anordnet, |
| Fig. 10 | eine vierte Ausführungsform der Erfindung in einer der Fig. 2 entsprechenden Ansicht, wobei Magneten in gleicher Anordnung auf beiden Seiten einer Trägerscheibe angeordnet sind, |
| Fig. 11 und 12 | Schnitte längs Linie C-D der Fig. 10 bei verschiedenen Ausführungsformen des Antriebs, |
| Fig. 13 | eine fünfte Ausführungsform der Vorrichtung in einer der Fig. 5 entsprechenden Ansicht, wobei auf beiden Seiten einer Trägerplatte Magneten in gleicher Anordnung vorgesehen sind, |
| Fig. 14 und 15 | Schnitte längs Linie C-D der Fig. 10 bei verschiedenen Ausführungsformen des Antriebs, |
| Fig. 16 | eine sechste Ausführungsform der Erfindung in der Draufsicht, wobei auf beiden Seiten einer Trägerscheibe Magneten in gleicher Anordnung vorgesehen sind, |
| Fig. 17 und 18 | Schnitte längs Linie C-D der Fig. 16 bei verschiedenen Ausführungsformen des Antriebs, und |
| Fig. 19 bis 23 | verschiedene Anwendungsbeispiele. |

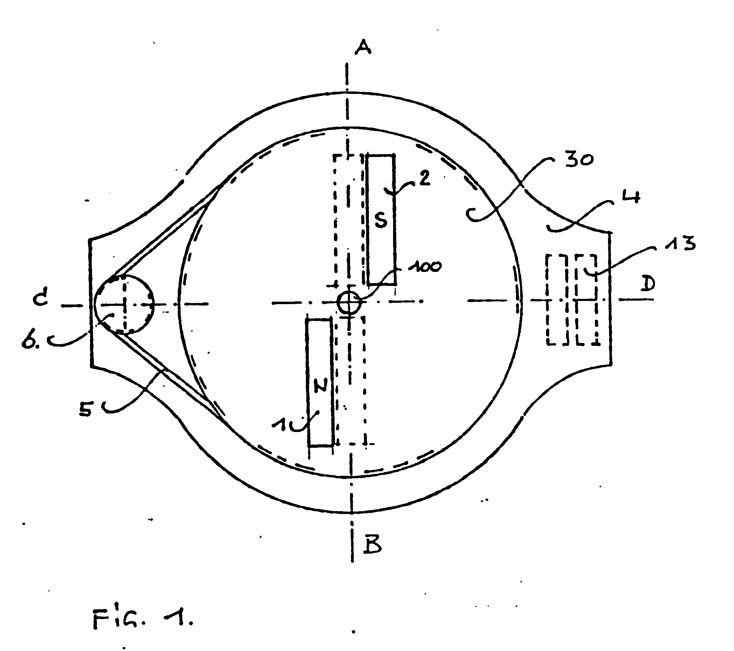

Gemäß Fig. 1 sind auf einer in einem Gehäuse 4 angeordneten um eine Achse 100 drehbaren Scheibe 30 zwei Permanentmagnete 1, 2 mit zur Scheibenebene senkrechter bzw. zur Drehachse 100 paralleler und untereinander entgegengesetzter Polarisation um 180° versetzt bezüglich der Drehachse 100 angeordnet. Die Lage der beiden Permanentmagnete 1, 2 ergibt sich, indem man sie aus der gestrichelt gezeichneten Lage um gleiche Beträge parallel zu sich selbst nach entgegengesetzten Seiten der Ebene A-B versetzt. Gleichbedeutend damit ist ein Verschwenken der beiden Magnete aus der Diametrallage um gleiche Winkel um gedachte radial außen liegende Achsen im gleichen Sinne, d.h. für beide Magneten im Uhrzeigersinn oder im Gegenuhrzeigersinn.

Die senkrecht zur Scheibenebene gemessene Höhe der Magneten ist gering gegenüber deren in Richtung der Scheibenebene gemessenen Längen- und Breitenabmessungen, d.h. gegenüber den Abmessungen der Polflächen. Es handelt sich also um magnetische Platten. Die Längenabmessung ist so gewählt, daß sich jeder Magnet im wesentlichen über den gesamten Radius der Scheibe erstreckt, während in Umfangsrichtung der Scheibe große freie Bereiche zwischen den Magneten vorhanden sind. Die bevorzugte Feldstärke, gemessen an den Polflächen der Permanentmagnete, beträgt vorzugsweise ca. 1000 Gauß, der Abstand zum zu behandelnden Objekt wird vorzugsweise zu 10 mm gewählt. Der Abstand zwischen den auf der Scheibe 30 befindlichen Permanentmagneten 1 und 2 und dem zu behandelnden Objekt wird vorzugsweise durch eine über der Scheibe angeordnete Platte aus amagnetischem und elektrisch nicht leitendem Material, beispielsweise Glas oder Kunststoff, bestimmt, wobei der Abstand der Platte zu der die Magnete tragenden Scheibe veränderbar sein kann. Zur Vermeidung von Feldverzerrungen werden die die Magnete tragende Scheibe 30 sowie das Gehäuse 4 ebenfalls aus amagnetischem und elektrisch nicht leitendem Material gewählt. Ebenso werden auch die Welle bzw. das Lager der drehbaren Scheibe 30 aus amagnetischem Werkstoff gewählt.

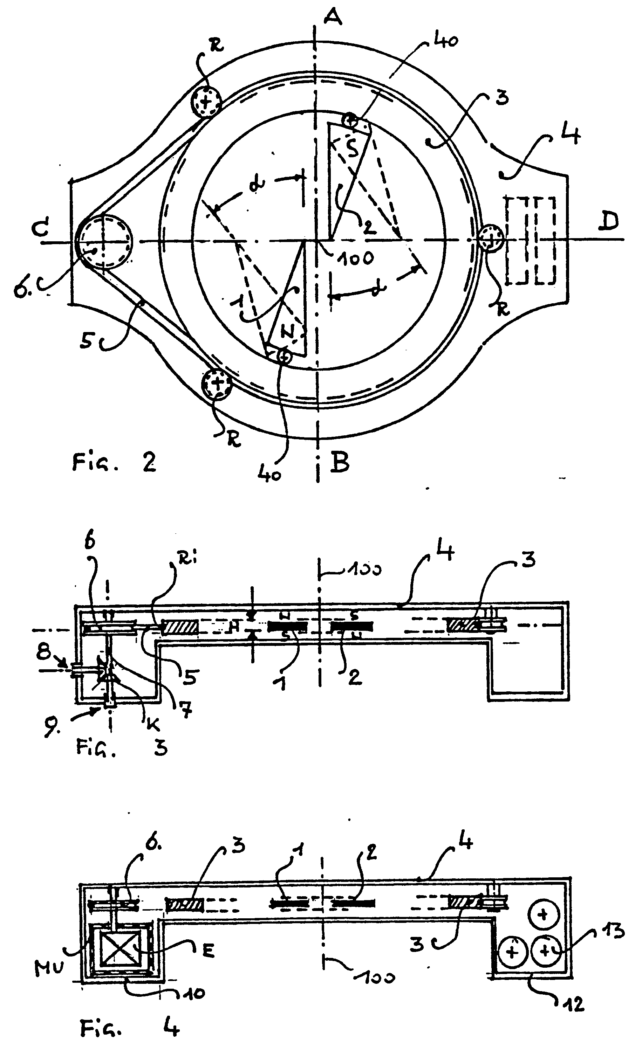

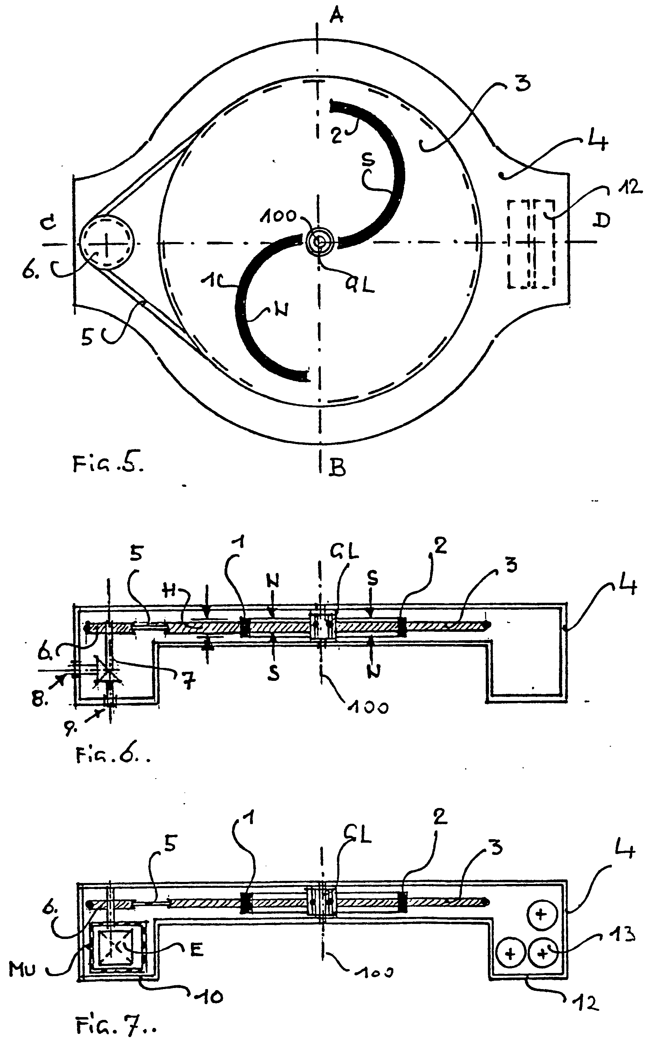

Fig. 2 zeigt eine weitere Ausführungsform der Vorrichtung. Der Träger für die wie bei der Ausführungsform der Fig. 1 entgegengesetzt gepolten Magnete 1 und 2 ist als Ring 3 ausgebildet. Die Permanentmagnete, die wiederum, wie sich aus den Fign. 3 und 4 ergibt, in Polarisationsrichtung, also in Richtung der Drehachse 100 eine geringe Höhe H haben, sind keilförmig ausgebildet und verjüngen sich vom Rand zur Mitte des Rings 3 hin. Die beiden Permanentmagnete 1 und 2 sitzen bezüglich der Drehachse um 180° versetzt in einer Anordnung, die man erhält, wenn man die beiden keilförmigen Magnete 1 und 2 aus einer diametralen Anordnung heraus um eine im Bereich ihrer Außenränder befindliche Achse um gleiche Winkel im Gegenuhrzeigersinn verschwenkt. Bei der Ausführungsform der Fig. 2 ist außerdem die Schwenklage der Magneten 1 und 2 veränderbar, wobei sie aus der mit durchgehenden Linien gezeigten Lage um einen zusätzlichen Winkel α im Gegenuhrzeigersinn bis in die gestrichelt gezeichnete Lage verschwenkbar sind. Die Schwenkachsen befinden sich auf dem Innenrand des Ringes 3 und sind bei 40 angedeutet. In jedem Fall ist die Symmetrie so, daß eine Drehung des Ringes um 180° um seine Drehachse die beiden Magneten ineinander überführt.

Magnete und Ring befinden sich, wie besser in den Fign. 3 und 4 zu sehen, in einem ganz geschlossenen Gehäuse 4 aus amagnetischem und elektrisch nicht leitendem Material. Die ebene Deckfläche des Gehäuses 4, an der das zu behandelnde Objekt zur Anlage kommt, bestimmt dabei gleichzeitig den Abstand desselben zu den Magneten. Dieser kann vorzugsweise 5 bis 10 mm betragen.

Der Antrieb des Ringes 3 erfolgt über einen um die Peripherie des Ringes 3 und eine zu diesem koaxiale Antriebsrolle 6 geführten Riemen 5. Ferner sind an der Peripherie des Ringes 3 drei Rollen R mit Ringnuten vorgesehen, die den Ring 3 und den Antriebsriemen 5 axial in Stellung halten. Die Antriebsrolle 6 wird über eine zur Ringachse parallele Welle 7 angetrieben, die ihrerseits über eine in ihrer Verlängerung liegende Welle 9, oder unter Zwischenschaltung eines Kegelradgetriebes K über eine senkrecht dazu verlaufende Welle 8 angetrieben werden kann, wobei wahlweise die eine oder andere der Wellen 8 und 9 mit einem Elektromotor verbunden sein kann. Der Elektromotor befindet sich außerhalb des Gehäuses 4 um Feldstörungen durch diesen nach Möglichkeit zu vermeiden.

Bei der in Fig. 4 gezeigten Abwandlung ist ein mit Mu-Metall Mu abgeschirmter Elektromotor E am Rand innerhalb einer Griffschale 10 des Gehäuses angeordnet, während Batterien bzw. Akkumulatoren 13 zu seiner Stromversorgung in einer diametral gegenüberliegenden Griffschale 12 angeordnet sind.

Die keilförmige Ausbildung der beiden Permanentmagnete 1 und 2 bewirkt ein Magnetfeld innerhalb der überstrichenen Kreisfläche, das bei um die Achse 100 drehenden Magneten in seinem zeitlichen Verlauf einigermaßen unabhängig vom Abstand zur Drehachse ist. Die außerradiale Anordnung der Magneten bewirkt eine therapeutisch zu berücksichtigende Asymmetrie der Magnetkräfte in Abhängigkeit vom Drehsinn des Rings 3 mit den daran angebrachten Permanentmagneten 1 und 2.

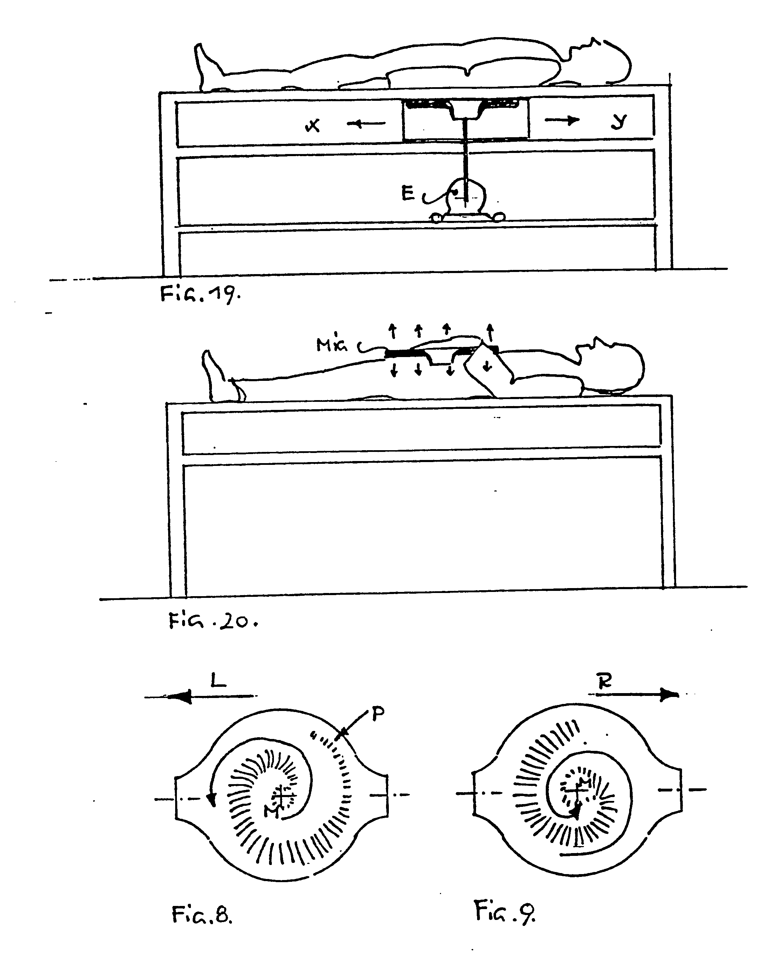

Diese Asymmetrie der Magnetkräfte zeigen die Fign. 8 und 9. Bei einer - bei Betrachtung gemäß Fig. 2 - Gegenuhrzeigersinndrehung ergibt sich ein spiralförmiges Auslaufen von auf dem Gehäuse liegendem ferromagnetischem Granulat von der Mitte zum Rand, während umgekehrt bei einer Drehung im Uhrzeigersinn das Granulat spiralförmig vom Rand zur Mitte läuft. Bei diesem Effekt zeigt sich eine Abhängigkeit vom Schwenkwinkel der beiden Permanentmagnete 1 und 2.

Ein nahezu radialer zentripetaler bzw. zentrifugaler Verlauf eines solchen Granulats zeigt sich bei der Ausführungsform der Fig. 5, bei der die Permanentmagnete - unter Beibehaltung ihrer zueinander entgegensetzten Polung parallel zur Drehachse - nicht keilförmig, sondern beide bogenförmig mit in der Drehebene liegender Krümmung ausgebildet sind. Die beiden Magnete liegen wiederum um 180° bezüglich der Drehachse versetzt, so daß sie zusammen eine S-Struktur ausbilden.

Bei der Ausführungsform der Fig. 5 sind die beiden Magnete 1 und 2 nicht innen an einem Ring 3 angebracht, sondern in eine auf einem amagnetischen Lager GL gelagerte Scheibe 30 eingelassen.

Das Einlassen der beiden Magneten 1, 2 in die Scheibe 30, das auch in Abwandlung der Ausführungsform der Fig. 1 vorgesehen sein kann, bzw. das Anbringen der beiden Magneten innen im Ring 3 ist so ausgeführt, daß sich für die Lage der Magnete symmetrische Verhältnisse bezüglich der Scheiben- oder Ringebene ergeben. In Verbindung mit der gewählten Ausbildung des Gehäuses so, daß über und unter der Scheibe bzw. dem Ring liegende Teile des Gehäuses ebenfalls symmetrisch zur Scheiben- oder Ringebene liegen, können die Vorrichtungen mit gleichen Verhältnissen für die Feldstärke am Applikationsort nach beiden Seiten hin angewandt werden. Ein Wenden der Vorrichtung kehrt dann ohne Umkehr der Drehrichtung des Motors die Felddynamik (zentrifugal - zentripetal) am Applikationsort um.

Möglichkeiten für den Antrieb der Scheibe ergeben sich aus den Fign. 6 und 7 und stimmen mit den in den Fign. 3 und 4 gezeigten überein.

Die Figuren 10 und 11 bzw. 10 und 12 zeigen eine von der Ausführungsform der Figuren 2 und 3 bzw. 2 und 4 abgeleitete doppelseitige Ausführungsform, bei der Magneten 1, 2 bzw. 1', 2' auf beiden Seiten einer Scheibe 30 angeordnet sind. Die Scheibe 30 ist hierbei aus einem magnetischen Werkstoff ausgebildet, um die Felder der zu beiden Seiten der Scheibe befindlichen Magneten 1, 2 bzw. 1', 2' gegeneinander abzuschirmen. Die Anordnung der Magneten 1, 2 bzw. 1', 2' auf den beiden Seiten der Scheibe 30 ist dabei so gewählt, daß sich bei Blick auf eine Seite der Scheibe 30 die auf dieser Seite vorgesehenen Magnete in der gleichen relativen Anordnung befinden, wie sie sich für die auf der anderen Seite der Scheibe befindlichen Magnete bei Blick auf diese andere Seite darstellt. Einen gegenseitige Verdrehung der beiden Anordnungen ist zulässig. Mit anderen Worten, durch Wenden der Scheibe 30 um die Achse C-D der Fig. 10 (und gegebenenfalls eine Drehung um die Drehachse 100) werden die auf der einen Seite der Scheibe befindlichen Magnete 1, 2 in die auf der anderen Seite der Scheibe befindlichen Magneten 1', 2' überführt.

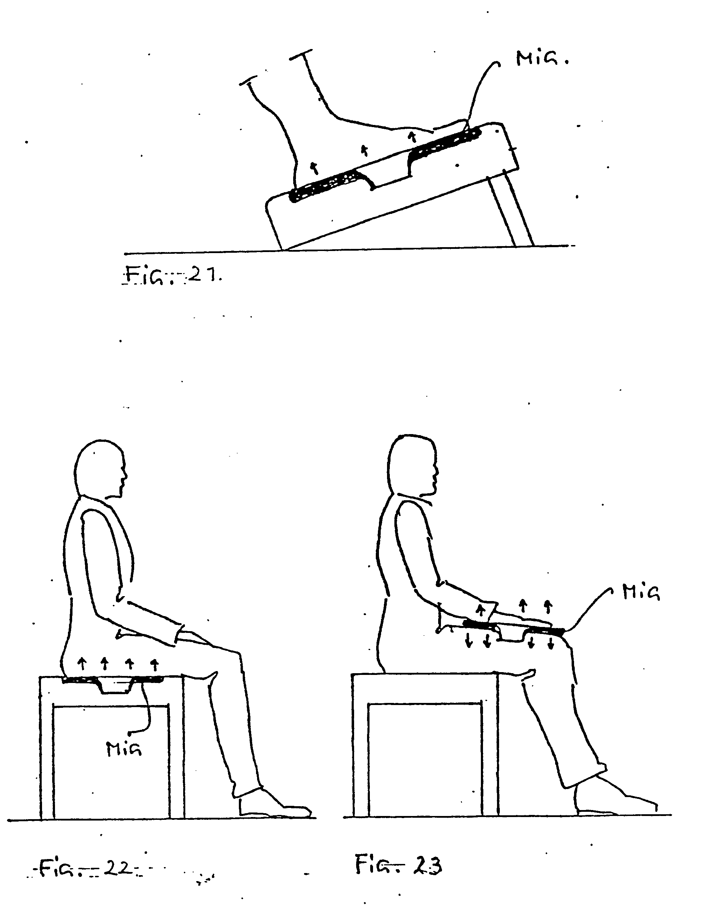

Bei einer Applikation der Vorrichtung von beiden Seiten her, wie sie beispielsweise in Fig. 23 dargestellt ist, wo der Patient die Vorrichtung auf den Oberschenkeln hält und die Hände darauf legt, ist bei einer solchen Anordnung der Magnete 1, 2 und 1', 2' erreicht daß sich auf beiden Seiten die gleiche Felddynamik, d.h. beidseits mit zentrifugaler oder beidseits mit zentripetaler Wirkung, je nach Drehrichtung der Scheibe, ergibt.

Gemäß Fig. 10 sind die Magnete 1, 2 und 1', 2' parallel zur Ebene der Scheibe 30 verschwenkbar (die Verschwenkbarkeit ist nur für die Magnete 1, 2 der einen Seite gestrichelt dargestellt).

Die Figuren 13 und 14 bzw. 13 und 15 zeigen eine Ausführungsform, die sich hinsichtlich der Anordnung der auf beiden Seiten der magnetisch abschirmenden Scheibe 30 befindlichen Magnete zu der Ausführungsform der Figuren 5 und 6 bzw.

5 und 7 genauso verhält wie die Ausführungsform der Figuren 10 und 11 bzw. 10 und 12 zu derjenigen der Figuren 2 und 3 bzw. 2 und 4.

Auch hier ist damit erreicht, daß sich nach beiden Applikationsrichtungen die gleiche jeweils nach innen wirkende oder jeweils nach außen wirkende Felddynamik ergibt.

Ohne daß dies im einzelnen dargestellt ist, kann auch die Ausführungsform der Fig. 1 so abgewandelt sein, daß Magneten in gleicher Relativanordnung zu beiden Seiten einer magnetisch abschirmenden Scheibe vorhanden sind.

Die Ausführungsform der Figuren 16 und 17 bzw. 16 und 18 sieht auf beiden Seiten der magnetisch abschirmenden Scheibe 30 geteilte keilförmige Magnete, auf der einen Seite 1.1 und 1.2 bzw. 2.1 und 2.2, und auf der anderen Seite 1.1' und 1.2' bzw. 2.1' und 2.2', vor, wobei jeweils die Teile mit gleicher Polarisationsrichtung nach entgegengesetzten Seiten in Bezug auf die in Fig. 16 durch die Achse A-B definierte Diametrallage und die radial innen liegenden Teile nach entgegengesetzten Seiten von A-B wie die radial außen liegenden Teile versetzt sind.

Auf der anderen Seite der Scheibe 30 ist im Sinne der Ausführungen zu den Magnetanordnungen der Figuren 10 und 13 die Magnetanordnung so gewählt, daß die Magnete der einen Seite durch eine gedachte Wendung (und gegebenenfalls Drehung um die Drehachse 100) der Scheibe in die Magnete der anderen Seite überführbar sind.

Anstelle der keilförmigen Magneten der Fig. 16 lassen sich auch Magnete konstanter Breite, also rechteckige Magnete verwenden.

Mit einer Ausführungsform wie in Fig. 16 ergeben sich zentrifugale und zentripetale Kraftwirkungen gleichzeitig.

Eine Vorrichtung kann auf der einen Seite geteilte und auf der anderen Seite ungeteilte Magnete aufweisen.

In allen dargestellten Ausführungsformen wurden zur Erzeugung der Polflächen in der gewünschten Anordnung Einzelmagnete dargestellt. Es ist aber im Prinzip auch möglich, gegennamige Polflächen als die beiden Enden eines einzigen Magneten zu erzeugen, der sich hinter der Ebene, in der diese Polflächen liegen, erstreckt.

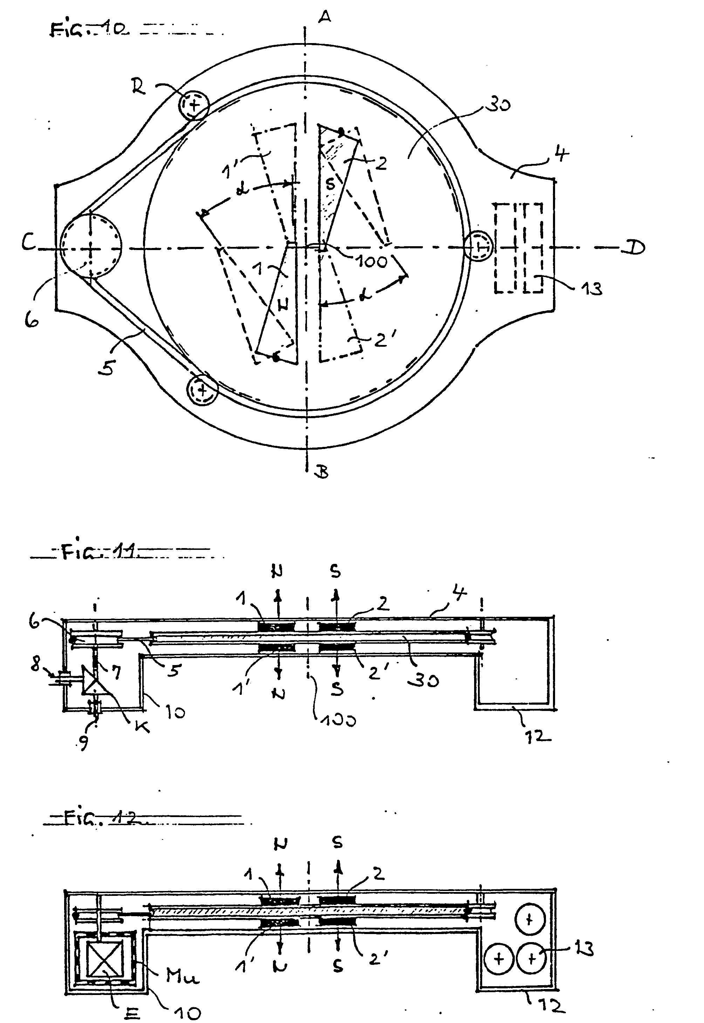

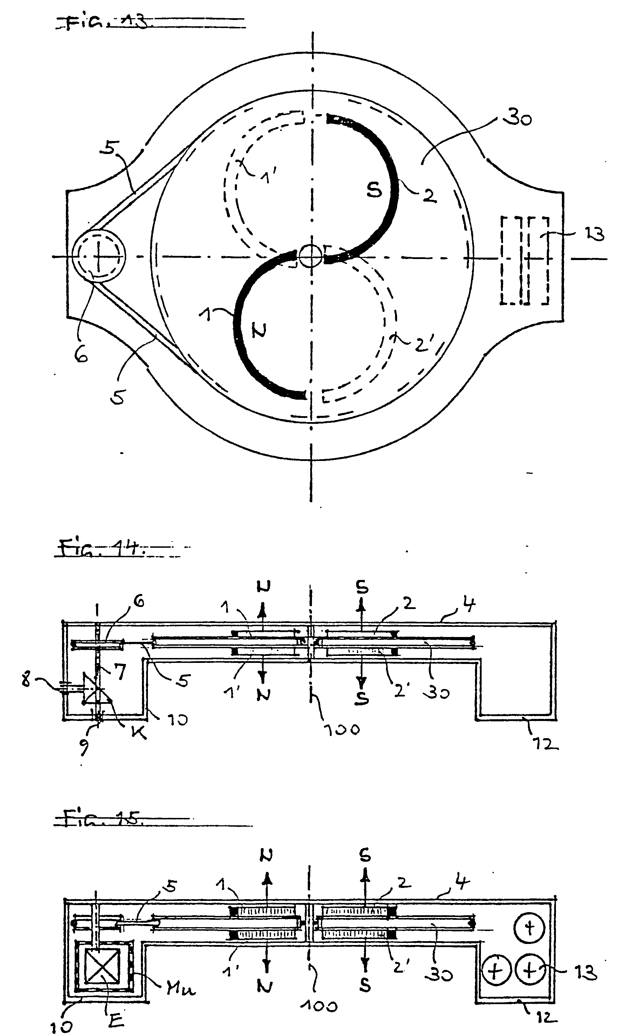

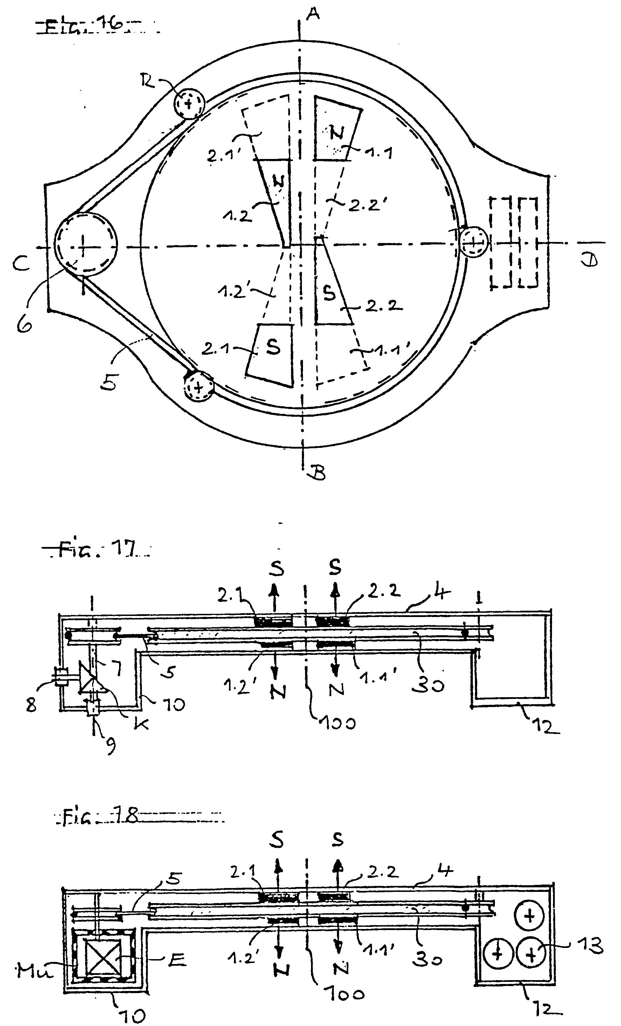

Die Fign. 9 bis 23 zeigen Anwendungsmöglichkeiten der Vorrichtung zur Erzeugung pulsierender Magnetfelder. In allen Fällen ist dafür Sorge getragen, daß die Scheiben bzw. Ringebene horizontal oder zumindest nahezu horizontal liegt. Die therapeutisch günstige Drehfrequenz des Magnetfelds ist von der Größenordnung 1 Hz. Die seitliche Anordnung des Motors ermöglicht die beidseitige Anwendung, wie sie sich aus den Fign. 20 und 23 ergibt. Bei der Anwendung gemäß Fig. 19 ist die gesamte Anordnung unter einer Liege, auf der sich der Patient befindet, verfahrbar.

Klinische Untersuchungen haben die therapeutischen Wirkungen wie folgt bestätigt:

Eine Drehrichtung der Vorrichtung mit zentripetaler Kraftwirkung (Drehung in Versetzungsrichtung, im Uhrzeigersinn bei den dargestellten Ausführungsformen) hat eine entspannende, sedierende Wirkung, die umgekehrte Drehrichtung eine tonisierende Wirkung.

Für die Drehung mit zentripetaler Kraftwirkung hat sich im einzelnen folgendes ergeben:

1. Eine deutliche Verbesserung bei Durchblutungsstörungen zeigte sich meist schon nach wenigen Minuten, wobei je nach Gesamtverfassung des Probanden dieser Effekt u. U. auch erst nach 15 - 20 Minuten eintrat. Diese positive 10-Veränderung wurde durch Infrarotaufnahmen eindeutig bestätigt.

2. Spannungszustände der Muskulatur wurden sehr rasch abgebaut. Dies wurde durch Untersuchungen der Beinmuskulatur mit Oberflächenmyographie statistisch signifikant verifiziert.

3. Serienuntersuchungen zeigten, daß unter dem Einfluß des Magnetimpulsgenerators der K/Ca - Quotient statistisch signifikant erhöht wird, was in Übereinstimmung mit der allgemeinen Beruhigung steht.

4. Bei spasmischen Darmbeschwerden, einer Störung der Peristaltik, bewirkt das Auflegen des Magnetimpulsgenerators auf den Bauch eine Normalisierung der Darmbewegung mit schwindenden Mißempfindungen.

## Patentansprüche

1. Vorrichtung zur Herstellung pulsierender Magnetfelder für therapeutische Zwecke mit
   einem um eine Achse (100) drehbaren Träger,
   einem oder mehreren auf dem Träger angeordneten Magneten (1,2; 1.1, 1.2, 2.1., 2.2), wobei der oder die Manete nach einer ersten Seite des Trägers weisende erste Polflächen entgegengesetzter Polarität aufweisen, die
   - paarweise vorhanden sind,
   - an ihrer Oberfläche eine zur Trägerfläche senkrechte Magnetisierungsrichtung aufweisen,
   - so geformt und angeordnet sind, daß durch eine Drehung des Trägers um 180° um seine Drehachse (100) das von ihnen ausgehende Magnetfeld unter Umkehr der Feldrichtung in sich übergeführt wird,
   - den geringeren Teil des von ihnen bei einer Drehung des Trägers um die Drehachse (100) überstrichenen Winkelbereichs einnehmen, und die Polflächen so angeordnet und geformt sind, daß das von ihnen in jedem Zeitpunkt erzeugte Feld keine radiale Symmetrie, d.h. keine die Drehachse enthaltende Ebene als Symmetrieebene hat.

2. Vorrichtung nach Anspruch 1, dadurch gekennzeichnet, daß die ersten Polflächen langgestreckt ausgebildet sind und sich mit ihrer Längserstreckung aus einem Bereich größerer Entfernung von der Drehachse (100) in einen Bereich geringerer Entfernung von der Drehachse erstrecken.

3. Vorrichtung nach Anspruch 2, dadurch gekennzeichnet, daß die ersten Polflächen rechteckigen Querschnitt haben (Fig. 1).

4. Vorrichtung nach Anspruch 2, dadurch gekennzeichnet, daß der Querschnitt der ersten Polflächen mit abnehmender Entfernung von der Drehachse (100) schmäler wird (Fig. 2).

5. Vorrichtung nach Anspruch 3, dadurch gekennzeichnet, daß die nicht radialsymmetrische Anordnung dadurch erreicht ist, daß die ersten Polflächen mit ihren Längsachsen nach entgegengesetzten seiten einer die Drehachse enthaltenden Ebene versetzt angeordnet sind.

6. Vorrichtung nach Anspruch 4, dadurch gekennzeichnet, daß die ersten Polflächen mit ihren Längsachsen nach entgegengesetzten Seiten einer die Drehachse (100) enthaltenden Ebene verschwenkt angeordnet sind und der Schwenkwinkel veränderbar ist.

7. Vorrichtung nach Anspruch 2, dadurch gekennzeichnet, daß die ersten Polflächen bogenförmig ausgebildet sind und zusammen eine S-Form bilden (Fig. 5).

8. Vorrichtung nach Anspruch 2, dadurch gekennzeichnet, daß weitere ersten Polflächen entgegengesetzter Polarität in einem anderen radialen Abstand zur Drehachse (100) als die ersten Polflächen entgegengesetzter Polarität vorgesehen sind und daß die ersten und die weiteren Polflächen in zueinander entgegengesetzten Richtungen außerhalb der radialen Symmetrie liegen.

9. Vorrichtung nach einem der Ansprüche 1 bis 7, dadurch gekennzeichnet, daß nach der anderen Seite des Trägers weisende zweite Polflächen vorgesehen sind, welche zueinander mit der gleichen Konfiguration und radialen Asymmetrie angeordnet sind wie die ersten Polflächen und daß zwischen den ersten und zweiten Polflächen eine magnetische Abschirmung vorgesehen ist.

10. Vorrichtung nach Anspruch 8, dadurch gekennzeichnet, daß nach der anderen Seite des Trägers weisende zweite und weitere zweite Polflächen vorgesehen sind, welche zueinander mit der gleichen Konfiguration und radialen Asymmetrie angeordnet sind wie die ersten und weiteren ersten Polflächen und daß zwischen den ersten und weiteren ersten Polflächen einerseits und den zweiten und weiteren zweiten Polflächen andererseits eine magnetische Abschirmung vorgesehen ist (Fig. 16).

11. Vorrichtung nach Anspruch 8, dadurch gekennzeichnet, daß nach der anderen Seite des Trägers weisende zweite Polflächen vorgesehen sind, welche zueinander mit der gleichen Konfiguration und radialen Asymmetrie angeordnet sind wie die ersten oder weiteren ersten Polflächen und daß zwischen den ersten und weiteren ersten Polflächen einerseits und den zweiten Polflächen andererseits eine magnetische Abschirmung vorgesehen ist.

## Claims

1. A device for producing pulsating magnetic fields for therapeutic purposes, with a substrate rotatable about an axis (100), with one or a plurality of magnets (1,2; 1.1, 1.2, 2.1, 2.2) disposed on the substrate, the magnet or magnets comprising, facing in a first side of the substrate, first pole faces of opposite polarity, which are
- provided in pairs,
- have on their surface direction of magnetisation perpendicular to the surface of the substrate
- so arranged and shaped that upon rotation of the substrate about its
- rotational axis (100) the magnetic field emanating from it is transferred into itself, with a reversal of the field direction,
- and occupy the lesser portion of the angular range swept by them upon rotation of the substrate about the axis, of rototion (100), and
the pole faces being so arranged and shaped that the field produced by them at any time has no radial symmetry, i. e. has no plane containing the rotational axle as the plane of symmetry.

2. A device according to claim 1, characterised in that the first pole faces are of elongated configuration and extend with their longitudinal extension from a region of greater distance from the rotational axis (100) into a region of lesser distance from the rotational axis.

3. A device according to claim 2, characterised in that the first pole faces are of rectangular cross-section (Fig. 1).

4. A device according to claim 2, characterised in that the cross-section of the first pole faces becomes narrower with decreasing distance from the rotational axis (100) (Fig. 2).

5. A device according to claim 3, characterised in that the non-radially-symmetrical arrangement is achieved in that the longitudinal axes of the first pole faces are offset towards opposite sides of a plane containing the rotational axis.

6. A device according to claim 4, characterised in that the first pale faces are arranged with their longitudinal axes pivoted towards opposite sides of a plans containing the rotational axis (100) and in that the pivot angle is variable.

7. A device according to claim 2, characterised in that the first pole faces are arcuate and together form an S-configuration (Fig. 5).

8. A device according to claim 2, characterised in that further first pole faces at opposite polarity are provided at a different radial distance from the rotational axis (100) than the first pole faces of opposite polarity and in that the first and the further first pale faces are disposed in relatively opposite directions outside of the radial symmetry.

9. A device according to one of claims 1 to 7, characterised in that second pole faces are provided which face in the other side of the substrate and which relative to one, another are disposed in the same configuration and radial asymmetry as the first pole faces and in that a magnetic shielding is provided between the first and second pole faces.

10. A device according to claim 8, characterised in that second and further second pole faces ore provided which relative to one another are disposed in the same configuration and radial asymmetry as the first and further first pole faces, and in that a magnetic shielding is provided between the first and further first pole faces an the one hand and the second and further second pole faces on the other (Fig. 16).

11. A device according to claim 8, characterised in that second pole faces are provided which face in the other side of the substrate and which relative to one another are disposed in the same configuration and radial asymmetry as the first or further first pole faces and in that a magnetic shielding is provided between the first and further first pole faces on the one hand and the second pale faces on the other.

## Revendications

1. Dispositif de production de champs magnétiques pulsatoires à des fins théraputiques, comprenant:
un support pouvant tourner autour d'un axe (100)
et un ou plusieurs aimants (1,2; 1.1, 1.2, 2.1, 2.2) disposés sur ce support,
ce ou ces aimants, qui comportent de premières surfaces polaires, de polarités opposées et tournées vers une première face du support,
- étant disposés par paire,
- offrant, sur leur surface, une direction d'aimantation perpendiculaire à la surface du support,
- ayant une forme et une disposition telles que, par une rotation du support de 180° autour de son axe de rotation (100), le champ magnétique qui en sort se transforme en lui-même lors d'une inversion de la direction de ce champ,
- et occupant la plus faible partie du domaine angulaire qu'ils balayent lors d'une révolution du support autour de l'axe de rotation (100),
tandis que les surfaces polaires ont une disposition et une forme telles que le champ qu'elles produisent à chaque instant ne possède pas de symétrie radiale, c'est-à-dire n'offre aucun plan contenant l'axe de rotation qui soit un

plan dé symétrie.

2. Dispositif suivant la revendication 1, caractérisé en ce que les premières surfaces polaires sont de forme allongée et s'étendent, dans le sens de leur longueur, d' une zone plus éloignée de l'axe de rotation (100) à une zone plus rapprochée de cet axe.

3. Dispositif suivant la revendication 2, caractérisé en ce que les premières surfaces polaires ont une section rectangulaire (Fig. 1).

4. Dispositif suivant la revendication 2, caractérisé en ce que la section des premières surfaces polaires devient plus étroite au fur et à mesure que diminue l'éloignement par rapport à l'axe de rotation (100) (Fig. 2).

5. Dispositif suivant la revendication 3, caractérisé en ce que la disposition sans symétrie radiale s'obtient par le fait que les premières surfaces polaires ont, par leurs axes longitudinaux, une disposition décalée en translation vers des côtés opposés d'un plan contenant l'axe de rotation.

6. Dispositif suivant la revendication 4, caractérisé en ce que les premières surfaces polaires ont, par leurs axes longitudinaux, une disposition décalée en rotation vers des côtés opposés d'un plan contenant l'axe de rotation (100) et en ce que l'angle de décalage en rotation est variable.

7. Dispositif suivant la revendication 2, caractérisé en ce que les premières surfaces polaires sont de forme incurvée et constituent ensemble un S (Fig. 5).

8. Dispositif suivant la revendication 2, caractérisé en ce qu'il est prévu d'autres premières surfaces polaires de polarité opposée et se trouvant, par rapport à l'axe de rotation (100), à une autre distance radiale que les premières surfaces polaires de polarité opposée et en ce que les premières et les autres premières surfaces polaires se trouvent en dehors de la symétrie radiale dans des directions opposées l'une à l'autre.

9. Dispositif suivant l'une quelconque des revendications 1 à 7, caractérisé en ce qu'il est prévu de secondes surfaces polaires tournées vers l'autre côté du support et qui sont agencées avec les mêmes configuration et asymétrie radiale entre elles que les premières surfaces polaires et en ce qu'il est prévu un écran antimagnétique entre les premières et secondes surfaces polaires.

10. Dispositif suivant la revendication 8, caractérisé en ce qu'il est prévu des secondes et d'autres secondes surfaces polaires tournées vers l'autre côté du support et qui sont agencées avec les mêmes configuration et asymétrie radiale entre elles que les premières et autres premières surfaces polaires et en ce qu'il est prévu un écran antimagnétique entre les premières et autres premières surfaces polaires d'une part et les secondes et autres secondes surfaces polaires d'autre part (Fig. 16).

11. Dispositif suivant la revendication 8, caractérisé en ce qu'il est prévu de secondes surfaces polaires tournées vers l'autre côté du support et qui sont agencées avec les mêmes configuration et asymétrie radiale entre elles que les premières ou autres premières surfaces polaires et en ce qu'il est prévu un écran antimagnétique entre les premières et autres premières surfaces polaires d'une part et les secondes surfaces polaires d'autre part.

FIG. 1.

0 159 043

Fig. 2

Fig. 3

Fig. 4

0 159 043

Fig. 5.

Fig. 6.

Fig. 7.

Fig. 19.

Fig. 20.

Fig. 8.

Fig. 9.

0 159 043

Fig. 10

Fig. 11

Fig. 12

9

FIG. 13

FIG. 14.

FIG. 15.

11

**Fig. 16**

**Fig. 17**

**Fig. 18**

Fig. 21.

Fig. 22.

Fig. 23